# EUROPÄISCHE PATENTSCHRIFT

(11) **EP 0 460 447 B1**
(45) Veröffentlichungstag und Bekanntmachung des Hinweises auf die Patenterteilung: **05.10.1994**
(21) Anmeldenummer: 91108140.4
(22) Anmeldetag: 21.05.1991
(51) Int. Cl.: A61B 17/16, B23B 47/28

(54) **Kompressions-Bohrlehre**
Compression drill guide
Calibre de perçage à compression

(30) Priorität: 06.06.1990 CH 1903/90
(43) Veröffentlichungstag der Anmeldung: 11.12.1991
(73) Patentinhaber: Synthes AG, Chur, CH-7002 Chur (CH)
(72) Erfinder: Gisin, Paul, CH-4437 Waldenburg (CH); Perren, Stephan M., CH-7260 Davos-Dorf (CH)
(74) Vertreter: Lusuardi, Werther Giovanni, Dr.

(56) Entgegenhaltungen:
- EP-A- 0 284 210
- EP-B- 0 173 267
- DE-A- 3 312 250
- DE-U- 8 813 655
- GB-A- 530 593
- US-A- 2 224 480
- US-A- 3 540 322
- US-A- 4 599 999

## Beschreibung

Die Erfindung bezieht sich auf eine Bohrlehre, gemäss dem Oberbegriff des Anspruchs 1.

Aus der EP-B1 0 173 267 ist eine Bohrlehre zur Anwendung bei der Kompressions-Osteosynthese bekannt, bei der die eigentliche Bohrbüchse exzentrisch angeordnet ist, so dass damit eine im voraus bestimmbare Kompressionswirkung erzeugt werden kann.

Nachteilig bei dieser Bohrlehre gemäss dem Stand der Technik ist die fehlende Berücksichtigung der Schaftgeometrie der zu implantierenden Schraube. Das plattenseitige Ende dieser bekannten Bohrlehre simuliert bloss die Geometrie des Kugelkopfes der zu implantierenden Schraube, was bei schiefwinkligen Bohrungen ungenügend sein kann.
Ein weiterer Nachteil besteht darin, dass die bekannte Bohrlehre nur die Ausführung von exzentrischen Bohrungen zulässt und somit die Verwendung einer weiteren Bohrlehre für neutrale Schraubenpositionierungen notwendig macht.
Mit Bohrlehren gemäss dem Stand der Technik ist es nicht möglich einen sphärischen Sitz im Schraubenloch zu realisieren, so dass keine Garantie besteht für eine spannungsfreie, d.h. von parasitären Zusatzbelastungen freie Osteosynthese.

Hier will die Erfindung Abhilfe schaffen. Der Erfindung liegt die Aufgabe zugrunde, eine universell verwendbare Bohrlehre zu schaffen, mit welcher sowohl neutrale, als auch versetzt angeordnete Bohrlöcher in einem weiten Winkelbereich gesetzt werden können.

Die Erfindung löst die gestellte Aufgabe mit einer Bohrlehre, welche die Merkmale des Anspruchs 1 aufweist.

Die durch die Erfindung erreichten Vorteile sind im wesentlichen darin zu sehen, dass dank der erfindungsgemässen Bohrlehre, bei welcher die Aussenform ihres distalen Teils genau der Aussenkontur der zu verwendenden Knochenschrauben entspricht, bereits während des Bohrvorgangs die spätere Position des Schraubenkopfes - relativ zum Bohrloch - mit grösster Genauigkeit gewählt werden kann. Sowohl die longitudinale Versetzung (bezogen auf die Längsachse der Knochenplatte) als auch die Neigung der Schraubenachse (bezogen auf die Senkrechte zur Plattenachse) ist, innerhalb eines Winkelbereiches von +/- 22°, mittels der erfindungsgemässen Bohrlehre im voraus exakt bestimmbar und innerhalb gewisser Grenzen begrenzbar.

Durch einen Federmechanismus ist es möglich die Geometrie des plattenseitigen Endes der Bohrlehre derart zu verändern, dass sowohl Bohrungen für Schrauben in neutraler als auch in versetzter Position ausgeführt werden können um nach Wunsch keine oder eine im voraus betragsmässig bestimmte Kompressionswirkung zu erzielen.

Im weiteren lässt sich die erfindungsgemässe Bohrlehre, dank des plattenseitig hervortretenden Endstücks des inneren, als Bohrbüchse dienenden Hohlzylinders, auch als Führungshülse verwenden. Zu diesem Zweck ist der innere Hohlzylinder vorzugsweise mit verschiedenen Längen auszuführen.

Da das plattenseitige Ende der erfindungsgemässen Bohrlehre genau der Form der Kopfunterseite der zu implantierenden Schrauben entspricht, ist es zweckmässig einen ganzen Satz von Bohrlehren mit abgestuften Dimensionen bereitzustellen, welche den verschiedenen Schraubendimensionen entsprechen.

Ein Ausführungsbeispiel der Erfindung, welches zugleich das Funktionsprinzip erläutert, ist in den Zeichnungen dargestellt und wird im folgenden näher beschrieben.
Fig. 1 stellt einen teilweisen Querschnitt durch die erfindungsgemässe Bohrlehre dar.
Fig. 2 stellt einen Längsschnitt durch eine unter Belastung des Federmechanismus in ein Kompressionsloch einer Knochenplatte eingesetzte Bohrlehre dar;
Fig. 3 stellt einen Längsschnitt durch eine Platten/Schrauben-Vorrichtung dar, welche durch Anwendung der Bohrlehrenpositionierung gemäss Fig. 2 erhalten wurde.
Fig. 4 stellt einen Längsschnitt durch eine ohne Belastung des Federmechanismus in ein Kompressionsloch einer Knochenplatte eingesetzte Bohrlehre dar;
Fig. 5 stellt einen Längsschnitt durch eine Platten/Schrauben-Vorrichtung dar, welche durch Anwendung der Bohrlehrenpositionierung gemäss Fig. 4 erhalten wurde;
Fig. 6 stellt einen Querschnitt durch eine erfindungsgemässe Bohrlehre zur Ausführung einer schiefwinkligen Bohrung dar;
Fig. 7 zeigt einen Querschnitt durch eine Platten-Schrauben-Vorrichtung, welche unter Verwendung der Bohrlehrenpositionierung gemäss Fig. 6 im Knochen befestigt wurde.
Fig. 8 stellt einen vertikalen Längsschnitt durch eine erfindungsgemässe Bohrlehre dar zur Ausführung einer zur Plattenlängsachse schrägwinkligen Bohrung;
Fig. 9 zeigt einen Längsschnitt durch die Platten-Schrauben-Vorrichtung gemäss Fig. 8; und
Fig. 10 stellt eine Ansicht der Bohrlehre dar mit einer am Haltegriff angebrachten zusätzlichen Bohrbüchse.

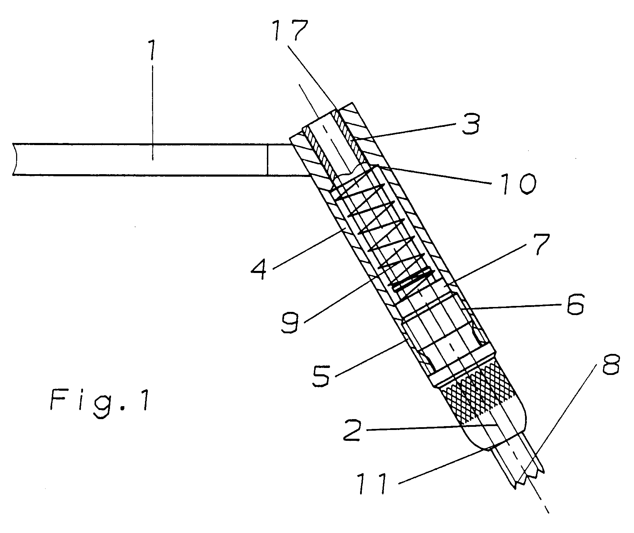

Die in Fig. 1 dargestellte Bohrlehre besteht aus einem, mit einem Haltegriff 1 versehenen, äusseren Hohlzylinder 4, in dem ein innerer Hohlzylinder 3 axial verschiebbar angeordnet ist. Die axiale Verschiebbarkeit des inneren Hohlzylinders 3 wird einerseits durch eine Schraubenverschlusshalterung 2, andererseits durch einen Federmechanismus 7,9,10 begrenzt.

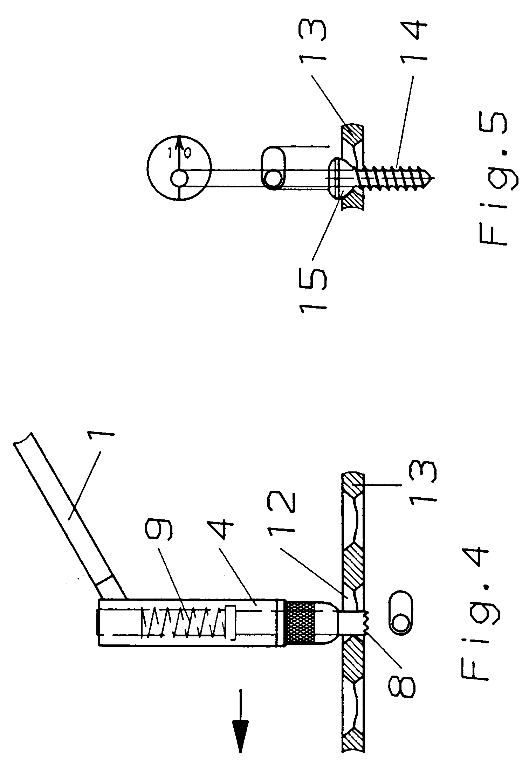

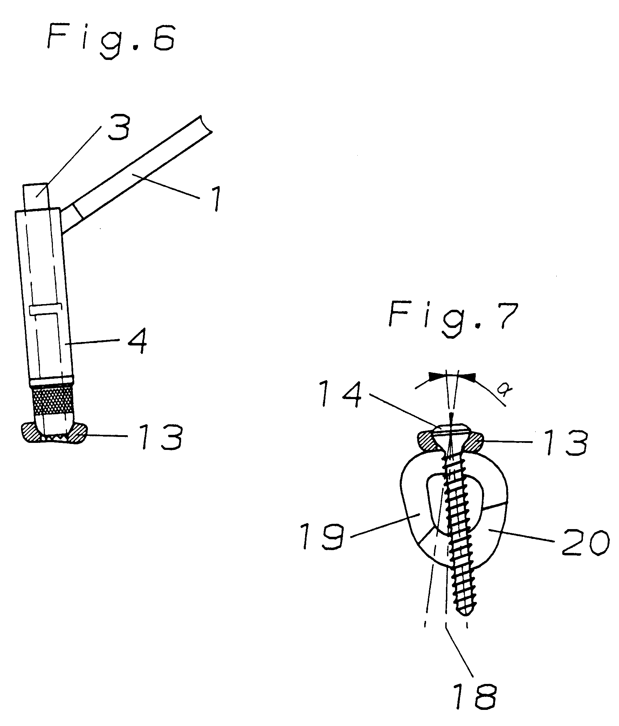

Die Schraubenverschlusshalterung 2 kann mittels eines proximal angeordneten Aussengewindes 6 in ein entsprechendes, distal angeordnetes Innengewinde 5 des äusseren Hohlzylinders 4 eingeschraubt werden. Die endständige Aussenform der Schraubenverschlusshalterung 2 entspricht dabei möglichst genau dem sphärische Profil der Kopfunterseite der zu verwendenden Kugelkopfschrauben. Im eingeschraubten, gebrauchsfertigen Zustand, wie er in Fig. 1 dargestellt ist, tritt das als gezackte Krone 8 ausgebildete distale Endstück des inneren Hohlzylinders 3 durch die distale Öffnung 11 der Schraubenverschlusshalterung 2 hervor und entspricht dabei in seiner Aussenform dem Schaftprofil der zu verwendenden Knochenschrauben (man vergleiche hierzu die Fig. 4 und 5).
Insgesamt entspricht somit die Aussenform des distalen Teils 2,8 der Bohrlehre der Aussenkontur 15 der zu verwendenden Knochenschrauben 14.

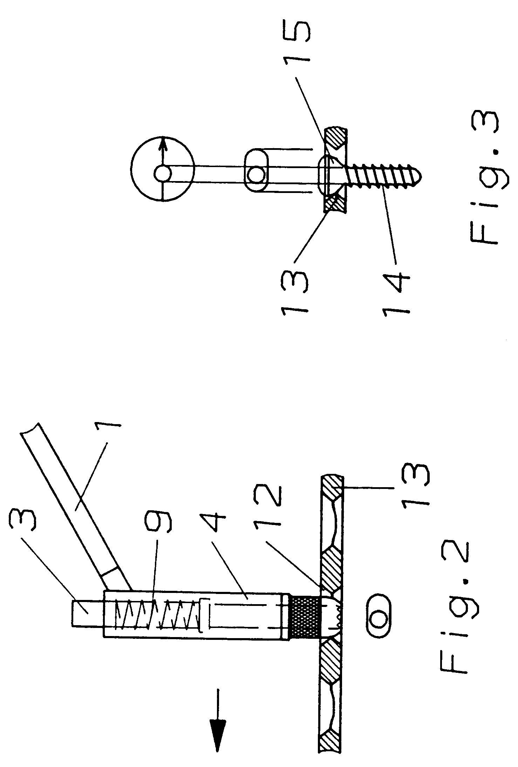

Der Federmechanismus 7,9,10 ist derart konstruiert, dass eine Spiralfeder 9 zwischen einem radialen Anschlag 7 des inneren Hohlzylinders 3 und einer radialen Verengung 10 des äusseren Hohlzylinders 4 eingespannt ist. Im belasteten Zustand (diese Position, bei welcher der hintere, proximale Teil des inneren Hohlzylinders 3 durch die proximale Öffnung 17 des äusseren Hohlzylinders 4 nach Aussen tritt ist in Fig. 2 dargestellt) ist die äussere Geometrie des plattenseitigen Endes 2 der Bohrlehre derart konfiguriert, dass bei dessen Einführung in das Bohrloch 12 der Knochenplatte 13 die zu setzende Knochenschraube 14, wie in Fig. 3 dargestellt, in eine neutrale Position kommt, bei der keine Kompressionswirkung ausübt wird. Der Chirurg kann diesen Vorgang dadurch optisch kontrollieren, dass er den aus der Bohrlehre heraustretenden, proximalen Teil des inneren Hohlzylinders 3 beachtet.

Dieser proximale Teil des inneren Hohlzylinders 3 kann ein optische Markierung, beispielsweise in Form farbiger Ringe, aufweisen (in der Zeichnung nicht dargestellt), welche dazu dient die Spannstellung der Bohrlehre abzustufen um dadurch verschieden grosse Kompressionswirkungen zu erzielen.

Wird jedoch auf die Bohrlehre (und damit auf die Krone 8 des inneren Hohlzylinders 3) kein Druck ausgeübt, so kann sich die Feder 9 entspannen, so dass das plattenseitige Ende 8 des inneren Hohlzylinders 3 um einen im voraus definierten Betrag aus der Schraubenverschlusshalterung 2 hervortritt. Durch das Hervortreten des als gezackte Krone 8 ausgebildeten distalen Endstücks des inneren Hohlzylinders 3 aus dem Inneren der Schraubenverschlusshalterung 2 verändert sich die plattenseitige Aussenkontur der Bohrlehre, so dass sie (wie in Fig. 4 und 5 dargestellt), gegenüber der neutralen Position im Kompressionsloch 12 der Knochenplatte 13, um einen im voraus bestimmten Betrag (hier 1,0 mm) in longitudinaler Richtung verschoben ist, was zu einer Kompressionswirkung der einzusetzenden Schraube 14 führt. Der Chirurg kann diesen Vorgang dadurch optisch kontrollieren, dass er den proximalen Teil der Bohrlehre beobachtet, wo der innere Hohlzylinder 3 in diesem Falle bündig mit dem äusseren Hohlzylinder 4 ist.

In Fig. 6 ist die Verwendung der erfindungsgemässen Bohrlehre zur Ausführung einer schiefwinkligen Bohrung dargestellt.
Die in Fig. 7 dargestellte unter Verwendung der Bohrlehrenpositionierung gemäss Fig. 6 eingesetzte Schraube 14 weist eine, gegenüber der axialen Symmetrieebene der Knochenplatte, um den Winkel α/2 geneigte Schraubenachse 18 auf.

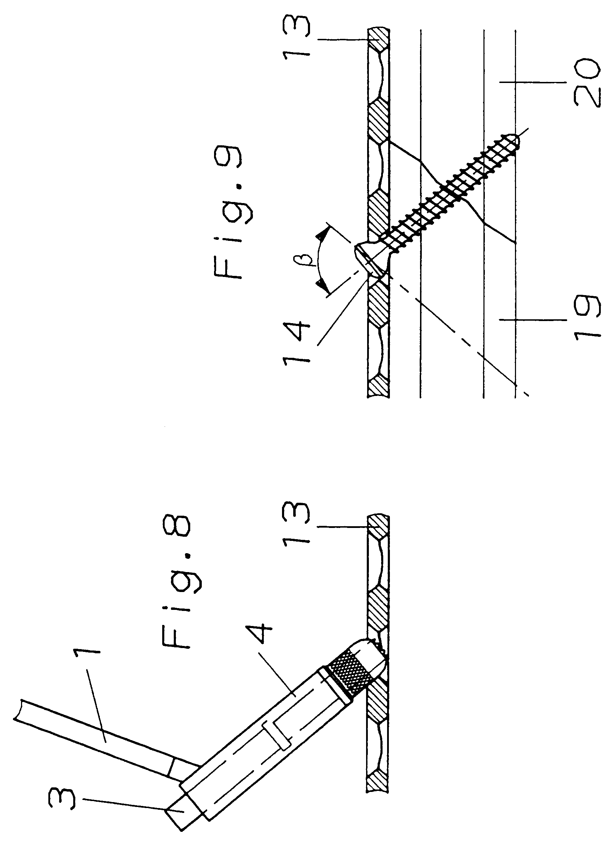

In Fig. 8 ist in einem Längsschnitt die Position der Bohrlehre dargestellt, wie sie sich bei der Ausführung einer zur Plattenlängsachse schrägwinkligen Bohrung ergibt.

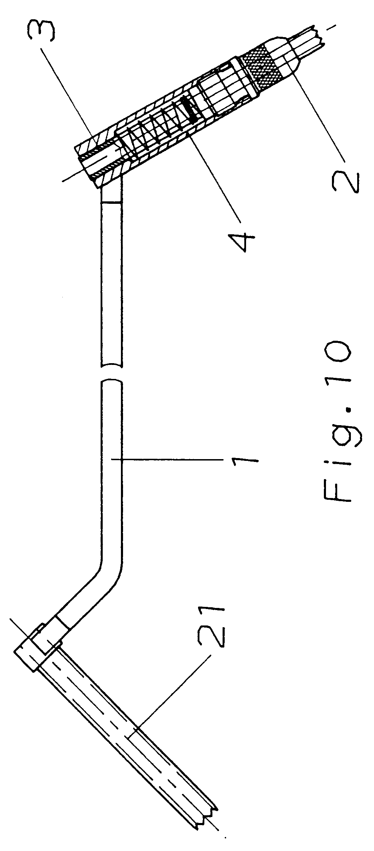

Fig. 9 zeigt die gleiche Schraube 14 gemäss Fig. 7, in der durch die Plattenlängsachse und den Röhrenknochen 19,20 verlaufenden Mitteleben (axiale Symmetrieebene der Knochenplatte), wobei die Knochenfragmente 19 und 20 von der Schraube 14 gegeneinander fixiert sind. In dieser Ebene weist die Schraube 14 einen Winkel von β/2 gegenüber der Orthogonalebene zur Plattenlängsachse auf.

Es sind kombinierte Winkelstellungen der Schraube 14 in seitlicher Richtung und in Längsrichtung innerhalb der angegebenen Winkelbereiche α und β möglich. Die Bohrlehre lässt jedoch dank ihrer Aussengeometrie nur Bohrungen innerhalb der vorgegebenen Inklinationsbereiche α, bzw. β zu, was die Sicherheit erhöht.

Bei der in Fig. 10 dargestellten bevorzugten Ausführungsform der erfindungsgemässen Bohrlehre ist am Haltegriff 1 eine zusätzliche Bohrbüchse 21 mit einem im Vergleich zum inneren Hohlzylinder 3 grösseren Innendurchmesser angebracht. Bei einem Innendurchmesser von 3,2 mm des inneren Hohlzylinders 3 (dieser sollte dem Innendurchmesser des jeweils zu verwendenden Kernlochbohrers entsprechen) beträgt der Innendurchmesser der zusätzlichen Bohrbüchse 21 4,5 mm. Damit ist es möglich Bohrlöcher vom Durchmesser 3,2 mm im proximalen Bereich der nahen Kortikalis aufzuweiten um die Applikationen von Knochenschrauben mit einer proximal glatten, stärker dimensionierten Schaftpartie zu gestatten.

Der Hauptvorteil der erfindungsgemässen Bohrlehre besteht darin, dass die mit dem Plattenloch 12 in Eingriff kommenden Teile der Bohrlehre exakt mit der Unterfläche 15 des Schraubenkopfes und dem Aussendurchmesser des Schraubengewindes übereinstimmen. Damit kann im Unterschied zu Bohrlehren gemäss dem Stand der Technik eine in jeder beliebigen Position geometrisch richtige Passung realisiert werden.

Ein weiterer Vorteil besteht darin, dass die Halbkugel 2 der Unterfläche der Bohrlehre, welche mit dem Plattenloch 12 in Eingriff kommt, durch das Hervortreten des inneren Hohlzylinders 3 zylindrisch verlängert ist und dadurch keine Schraubenstellung realisiert werden kann, die beim Eindrehen der Schraube temporär durch die Kante des Plattenlochs 12 eine Seitenverschiebung erzwingt. Seitliche Kräfte belasten den Knochen zusätzlich und können dazu führen, dass die dadurch entstehenden Torsionsmomente die vorläufig adaptierten Knochenfragmente 19,20 gegeneinander verschieben oder intakte Knochenteile parasitären Spannungen ausgesetzt werden.
Ein weiter Vorteil der erfindungsgemässen Bohrlehre besteht darin, dass mit ihr je nach Eindrücken des teleskopischen, als gezackte Krone 8 ausgebildete distale Endstück des inneren Hohlzylinders 3 graduell unterschiedliche Kompressionswege erreicht werden können und dies in jeder beliebigen Neigung der Schraube 14.
Ebenfalls vorteilhaft ist sicher der Umstand, dass die Teleskopeinrichtung 3,7,9,10 und ihre geometrische Ausführung mit einer Halbkugel 2 es gestattet, eine Kompression überhaupt auszuüben, ohne dass die Bohrlehre gewechselt werden muss, wie dies bei Bohrlehren gemäss dem Stand der Technik erforderlich ist.
Ein letzter Vorteil ist darin zu erblicken, dass die erfindungsgemässen Bohrlehre als Steckbohrbüchse verwendet werden kann um die Plattenlage genau auf die Zugschraubenlage auszurichten und um eine präzise und spannungsfreie Osteosynthese zu gewährleisten.

## Patentansprüche

1. Bohrlehre zum Bohren von Platten-Schrauben-Löchern in zu stabilisierende Knochenfragmente mittels einer Vorrichtung, welche eine Kompressionsknochenplatte (13) mit darin ausgebildeten durchgehenden Löchern (12) und dazu passenden Kugelkopfschrauben (14) umfasst, dadurch gekennzeichnet, dass die Bohrlehre einen zur Führung des Bohrers bestimmten, in einem äusseren Hohlzylinder (4) mittels eines Federmechanismus (7,9,10) koaxial verschiebbar angeordneten, inneren Hohlzylinder (3) aufweist, wobei das plattenseitige Ende (2) des äusseren Hohlzylinders (4) und das, bei unbelastetem Federmechanismus daraus herausragende plattenseitige Ende (8) des inneren Hohlzylinders (3) im wesentlichen der sphärischen Form der Kopfunterseite (15) und des Schaftes (16) der zu implantierenden Schrauben (14) entspricht.

2. Bohrlehre nach Anspruch 1, dadurch gekennzeichnet, dass das aus dem plattenseitigen Ende (2) des äusseren Kohlzylinders (4) herausragende plattenseitige Ende (8) des inneren Kohlzylinders (3) derart bemessen ist, dass bei unbelastetem Federmechanismus (7,9,10) ein Bohrloch erzeugt wird, welches gegenüber der neutralen Position im Kompressionsloch (12) um einen definierten Betrag in longitudinaler Richtung verschoben ist.

3. Bohrlehre nach Anspruch 1 oder 2, dadurch gekennzeichnet, dass die Bohrlehre einen Haltegriff (1) aufweist.

4. Bohrlehre nach Anspruch 3, dadurch gekennzeichnet, dass am Haltegriff (1) eine zusätzliche, als Gewebeschutzhülse ausgebildete Bohrbüchse (21) mit einem im Vergleich zum inneren Hohlzylinder (3) grösseren Innendurchmesser angebracht ist.

5. Bohrlehre nach einem der Ansprüche 1 bis 4, dadurch gekennzeichnet, dass der proximale Teil des inneren Hohlzylinders (3) eine optische Markierung, vorzugsweise in Form farbiger Ringe, aufweist.

## Claims

1. Drill guide for drilling plate screw holes in fractured bone segments to be stabilized my means of a device comprising a compression bone plate (13) having through-going screw holes (12) and round-headed screws (14) adapted to fit said screw holes (12), characterized in that said drill guide comprises an inner hollow cylinder (3) coaxially slidingly positioned within a hollow outer cylinder (4) by means of spring mechanism (7,9,10), said inner hollow cylinder (3) being designed to guide a drill, whereby the plate-side end (2) of said hollow outer cylinder (4) and the plate-side end (8) of said hollow inner cylinder (3), said latter plate-side end (8) projecting beyond the plate side end (2) of the said hollow outer cylinder (4) when the spring mechanism (7,9,10) is unloaded, having, respectively, essentially the spherical form of the underside (15) of the screw head and of the form of the shank (16) of the screws (14) to be implanted.

2. Drill guide according to claim 1, characterized in that the plate-side end (8) of said hollow inner cylinder (3), which projects from the plate-side end (2) of said hollow outer cylinder (4), is of such size that when the spring mechanism (7,9,10) is unloaded a drill hole may be created that is displaced longitudinally for a specific distance with respect to the neutral position in said screw hole (12).

3. Drill guide according to claim 1 or 2, characterized in that the drill guide comprises a support arm (1).

4. Drill guide according to claim 3, characterized in that an additional drill bushing (21) for use as a tissue protective sleeve is provided on said support arm (1) said drill bushing (21) having a larger internal diameter than said hollow inner cylinder (3).

5. Drill guide according to one of the claims 1 - 4, characterized in that the proximal segment of said hollow inner cylinder (3) is provided with an optical marking, preferably in the form of coloured rings.

## Revendications

1. Gabarit de forage pour le forage de trous pour vis et plaques dans des fragments d'os à stabiliser au moyen d'un dispositif, qui comporte une plaque de compression pour os (13) avec des trous (12) traversants qui y sont pratiqués et des vis à tête sphérique (14) qui s'adaptent à ces derniers, caractérisé en ce que le gabarit de forage présente un cylindre creux intérieur (3), défini pour le guidage du foret, disposé dans un cylindre creux extérieur (4) de manière à pouvoir être déplacé coaxialement au moyen d'un mécanisme à ressort (7, 9, 10), l'extrémité (2), située côté plaque, du cylindre creux extérieur (4) et l'extrémité (8), située côté plaque, du cylindre creux intérieur (3), qui en déborde lorsque le mécanisme à ressort n'est pas en charge, correspondent essentiellement à la forme sphérique de la face inférieure (15) de la tête et à la tige (16) des vis (14) à implanter.

2. Gabarit de forage selon la revendication 1, caractérisé en ce que l'extrémité (8), située côté plaque, du cylindre creux extérieur (4), débordant hors de l'extrémité (2), située côté plaque, du cylindre creux intérieur (3), est dimensionnée de telle sorte que, lorsque le mécanisme à ressort (7, 9, 10) n'est pas en charge, on crée un trou foré qui, par rapport à la position neutre dans le trou de compression (12), est déplacé d'une valeur définie dans la direction longitudinale.

3. Gabarit de forage selon la revendication 1 ou 2, caractérisé en ce que le gabarit de forage présente une poignée de maintien (1).

4. Gabarit de forage selon la revendication 3, caractérisé en ce que sur la poignée de maintien (1) est installée une douille de forage (21) supplémentaire configurée comme douille de protection des tissus, présentant un diamètre intérieur plus grand que celui du cylindre creux intérieur (3).

5. Gabarit de forage selon l'une des revendications 1 à 4, caractérisé en ce que la partie proximale du cylindre creux intérieur (3) présente un marquage visuel, de préférence sous la forme d'anneaux colorés.
